# EUROPEAN PATENT APPLICATION

(11) **EP 2 783 627 A1**
(43) Date of publication of application: **01.10.2014**
(21) Application number: 11878647.4
(22) Date of filing: 28.12.2011
(51) Int. Cl.: A61B 5/0402, A61B 7/00

(54) **DIAGNOSTIC DEVICE**

(71) Applicant: University of Tsukuba, Tsukuba-shi, Ibaraki 305-8577 (JP); Cyberdyne Inc., Ibaraki 305-0818 (JP)
(72) Inventor: SANKAI, Yoshiyuki, Tsukuba-shi Ibaraki 305-8577 (JP); NAKATA, Kin-ichi, Tsukuba-shi Ibaraki 305-0818 (JP)
(74) Representative: Regimbeau
(86) International application number: PCT/JP2011/080505
(87) International publication number: WO 2013/099020

(57) **Abstract**

A diagnostic apparatus includes a stethoscope, an electrocardiac detector, a control unit, and an external unit. A sound detector is a chest piece to make contact with a patient's skin, and includes a first detector to detect a relatively high frequency and a second detector to detect a relatively low frequency. The electrocardiac detector includes electrodes to detect potentials associated with heart beats, and is provided on each of the first and second detectors. The control unit converts electrocardiac signals detected by the electrocardiac detector into radio signals that are transmitted to the external unit. The external unit is a portable terminal apparatus, and includes a display unit formed by a liquid crystal panel on a front surface thereof, an operation part including operation buttons and ten-key, and patient selection buttons to select reading of data of patients.

## Description

### TECHNICAL FIELD

The present invention relates to diagnostic apparatuses, and more particularly to a diagnostic apparatus configured to make a diagnosis using cardiac sound and electrocardiogram.

### BACKGROUND ART

For example, when a physician diagnosis a patient, a simplified judgment may be made to determine body abnormalities by using a stethoscope to listen to cardiac sound from the patient's heart beat, respiratory sound from the patient's lungs, or the like. In addition, an x-ray examination, an electrocardiogram measurement, or the like may suitably be carried out according to patient's responses to the physician's questions and results of the diagnosis using the stethoscope.

A conventional diagnostic apparatus (for example, refer to Patent Document 1) may detect body sounds by providing a microphone at a sound collecting part of the stethoscope, and process audio signals output from the microphone into high-quality signals so that the body sounds are easy to hear.

### PRIOR ART DOCUMENTS

### PATENT DOCUMENTS

Patent Document 1: Japanese Laid-Open Patent Publication No. 2003-588

### DISCLOSURE OF THE INVENTION

### PROBLEM TO BE SOLVED BY THE INVENTION

However, although the conventional diagnostic apparatus enables the physician to listen to the body sounds detected by the stethoscope with a high quality when diagnosing the patient's symptoms, the patient is moved to another room where an electrocardiograph is set up in a case in which the cardiac sound includes an abnormality. The physician observes the patient's electrocardiogram measured by the electrocardiograph, in order to confirm whether the patient's cardiac valve operation includes an abnormality. For this reason, in the case in which the abnormality in the patient's heart is suspected, the electrocardiogram is measured without making the diagnosis solely based on the cardiac sound, and it takes time to diagnose the heart. In addition, when the abnormality is also confirmed from the electrocardiogram, an x-ray photography or a CT (Computed Tomography) scan is carried out, in order to make the diagnosis for specifying the cause of the heart abnormality.

On the other hand, when the physician makes a judgment on whether the measurement by a high-precision electrocardiograph using a 12-lead electrocardiograph, an electrogastrogram, an ambulatory or Holter electrocardiograph, or the like is necessary, it is difficult in many cases to make the judgment based solely on the cardiac sound detected by the existing stethoscope. Hence, there is a problem in that, to be on the safe side, the examination must be carried out with a high precision that is higher than actually required.

Accordingly, in view of the above, it is one object of the present invention to provide a diagnostic apparatus that can solve the problem described above.

### MEANS OF SOLVING THE PROBLEM

In order to solve the problem described above, the present invention may include the following means.
(1) A diagnostic apparatus of the present invention includes:
   a stethoscope including a sound detector that detects body sounds, a tube having one end thereof communicating to the sound detector, and a pair of ear tubes branching from another end of the tube, and configured to detect the body sounds from end parts of the pair of ear tubes;
   an electrocardiac detector, provided on the sound detector, and configured to detect potentials associated with heart beats;
   a control unit, provided on the stethoscope, and configured to convert the potentials detected by the electrocardiac detector into radio signals and transmit the radio signals; and
   an external unit, provided separately from the stethoscope, and configured to receive the radio signals transmitted from the control unit and display waveforms based on the radio signals.
(2) The electrocardiac detector and the control unit of the present invention are provided on a mounting base that is detachably mounted on the sound detector.
(3) The control unit of the present invention includes
   a radio transmitter configured to convert the potentials detected by the electrocardiac detector into the radio signals; and
   a battery configured to supply power to the radio transmitter.
(4) The external unit of the present invention is formed by a portable terminal apparatus including a radio receiver configured to receive the radio signals transmitted from the radio transmitter;
   a storage configured to store a change in the potentials from the radio receiver; and
   a display unit configured to display the waveforms based on the change in the potentials.
(5) The sound detector of the present invention includes
   a first detector having a diaphragm configured to detect a high frequency of the body sounds; and
   a second detector having a rubber member, provided on a peripheral edge part of the sound detector, and configured to detect a low frequency of the body sounds,
   wherein the first detector and the second detector are selectable, and
   wherein the electrocardiac detector is provided on each of the first detector and the second detector.
(6) A diagnostic apparatus of the present invention includes:
   a stethoscope unit including a sound detector configured to detect body sounds; and
   a terminal apparatus, provided separately from the stethoscope unit, and configured to receive radio signals transmitted from the stethoscope unit and display waveforms based on the radio signals and cardiac sound signals,
   wherein the stethoscope unit includes an electrocardiac detector configured to detect potentials associated with heart beats, a microphone configured to detect cardiac sound, and a radio transmitter configured to convert the potentials detected by the electrocardiac detector and the cardiac sound detected by the microphone into radio signals and transmit the radio signals to the terminal apparatus.
(7) The stethoscope unit of the present invention includes
   a storage configured to store the potentials detected by the electrocardiac detector and the cardiac sound signals detected by the microphone; and
   a battery configured to supply power to the radio transmitter.
(8) The terminal apparatus of the present invention includes
   a communication unit including a radio receiver configured to receive the radio signals of the potentials and the cardiac sound signals transmitted from the radio transmitter of the stethoscope unit;
   a storage configured to store a change in the potentials and the cardiac sound signals from the communication unit; and
   a display unit configured to display waveforms based on the change in the potentials detected by the electrocardiac detector and the cardiac sound signals detected by the microphone.

### EFFECTS OF THE INVENTION

According to the present invention, when the control unit provided in the stethoscope transmits the radio signals of the potentials associated with the heart beats, the waveforms based on the radio signals are displayed on the external unit that is provided separately from the stethoscope, and thus, it is possible to confirm the electrocardiograph while listening to the patient's heart sound using the stethoscope, to thereby improve the diagnostic efficiency and reduce the diagnostic time, and provide information that helps judgment as to whether a more detailed examination is required.

In addition, according to the present invention, when the control unit provided in the stethoscope unit transmits the radio signals of the potentials associated with the heart beat, the waveforms based on the radio signals are displayed on the external unit that is provided separately from the stethoscope unit, and for example, the heart sound and the electrocardiograph of the patient detected by the stethoscope unit can be transmitted with respect to the physician located at a remote location, even when the patient is located at an isolated island or mountain area with no physician, to enable diagnosis by the physician at the remote location, and improve the diagnostic efficiency by enabling operation by the patient or a helper who helps the patient, who may be unfamiliar with operation of medical equipment.

### BRIEF DESCRIPTION OF THE DRAWINGS

FIG. 1 is a perspective view illustrating one embodiment of a diagnostic apparatus according to the present invention;
FIG. 2 is a block diagram illustrating a system configuration of the diagnostic apparatus;
FIG. 3A is a longitudinal section of a sound detector in a state in which a diaphragm of a first detector makes contact with the patient's skin;
FIG. 3B is a bottom view illustrating an arrangement of the diaphragm and electrodes of the sound detector;
FIG. 3C is a plan view illustrating an arrangement of a rubber member and the electrodes at a peripheral edge part of the sound detector;
FIG. 3D is a longitudinal section of the sound detector in a state in which the rubber member of the sound detector makes contact with the patient's skin;
FIG. 4 is a cross sectional view illustrating detection points when the sound detector makes contact with a periphery of a chest part;
FIG. 5 is a diagram illustrating waveforms displayed on a display unit of an external unit;
FIG. 6 is a diagram illustrating a state in which a physician makes a diagnosis using the diagnostic apparatus of the present invention;
FIG. 7 is a block diagram illustrating a system configuration of a modification 1;
FIG. 8 is a longitudinal section of a stethoscope unit of the modification 1;
FIG. 9 is a bottom view illustrating an end surface of the stethoscope unit of the modification 1;
FIG. 10 is a diagram illustrating waveforms of signals detected by the stethoscope unit of the modification 1;
FIG. 11 is a diagram schematically illustrating a state in which the physician makes the diagnosis using the diagnostic apparatus of the modification 1;
FIG. 12 is a perspective view illustrating a state in which the electrocardiac unit of a modification 2 is separated from a stethoscope;
FIG. 13A is a plan view of the stethoscope unit of the modification 2 viewed from above;
FIG. 13B is a bottom view of the stethoscope unit of the modification 2 viewed from below;
FIG. 14 is a perspective view illustrating a state in which the electrocardiac unit of the modification 2 is connected to the stethoscope;
FIG. 15 is a plan view illustrating the state in which the stethoscope unit of the modification 2 is connected to the stethoscope;
FIG. 16A is a longitudinal section along a line A-A in FIG. 15;
FIG. 16B is a longitudinal section along a line B-B in FIG. 15;
FIG. 17A is a perspective view illustrating a state in which an electrocardiograph unit of a modification 3 is separated from the stethoscope; and
FIG. 17B is a longitudinal section illustrating a cross section of a part of the stethoscope unit of the modification 3.

### MODE OF CARRYING OUT THE INVENTION

A description will hereinafter be given of embodiments of the present invention with reference to the drawings.

### Embodiment 1

### [Configuration of Diagnostic Apparatus]

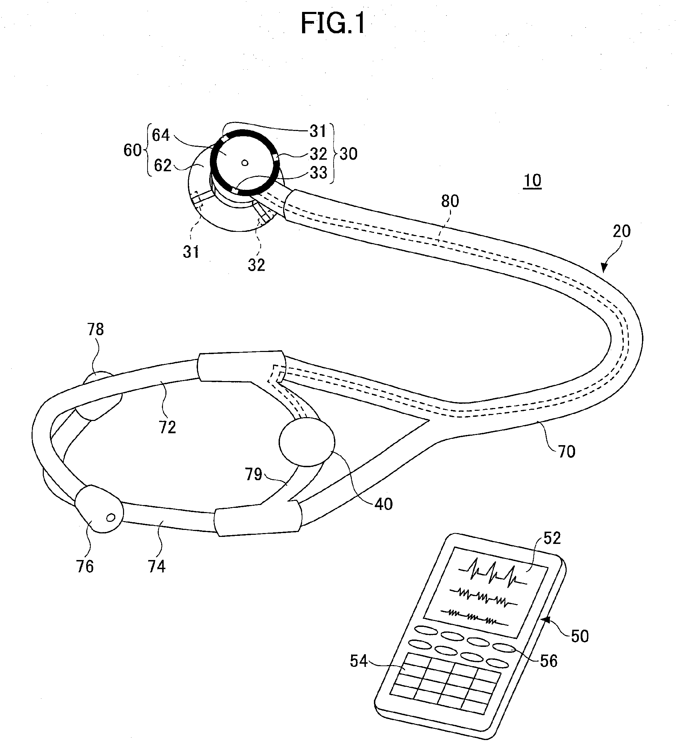

FIG. 1 is a perspective view illustrating one embodiment of a diagnostic apparatus according to the present invention. As illustrated in FIG. 1, a diagnostic apparatus 10 includes a stethoscope 20, an electrocardiac detector 30, a control unit 40, and an external unit 50. The stethoscope 20 includes a sound detector 60, a tube 70 having one end thereof communicating to the sound detector 60, and a pair of ear tubes 72 and 74 branching from the other end of the tube 70. A physician can insert ear tips 76 and 78 that are attached to end parts of the ear tubes 72 and 74, into the physician's external acoustic meatus, in order to listen to sounds detected from a part to which the sound detector 60 makes contact.

The sound detector 60 forms a chest piece that detects body sounds propagating through a patient's body, by making contact with the patient's skin. The sound detector 60 includes a first detector 62 that detects a relatively high frequency (for example, 200 Hz or higher), and a second detector 64 that detects a relatively low frequency (for example, 200 Hz or lower).

The electrocardiac detector 30 includes electrodes 31 through 33 for detecting potentials associated with heart beats, that are provided on each of the first and second detectors 62 and 64. The electrocardiac detector 30 of this embodiment measures potential vectors in each direction associated with the heart beats according to the bipolar lead type system which will be described later, and outputs waveform data of an electrocardiogram.

The control unit 40 is provided at an intermediate part of a communication tube 79 that communicates to the pair of branching ear tubes 72 and 74. The control unit 40 is electrically connected to each of the electrodes 31 through 33 of the electrocardiac detector 30 via wires 80, and as will be described later, the control unit 40 converts electrocardiac signals detected by the electrocardiac detector 30 into radio signals that are sent to the external unit 50.

The external unit 50 is a portable terminal apparatus that may be put into a pocket of a jacket or the like. The external unit 50 includes a display unit 52 formed by a liquid crystal panel on a front surface thereof, an operation part 54 including a plurality of operation buttons and ten-key, and a plurality of (8 buttons in FIG. 1) patient selection buttons 56 to select reading of data of each of the patients.

Accordingly, the physician can place the sound detector 60 of the stethoscope 20 to make contact with the patient, and listen to the body sounds (including heart beat and respiratory sound), while displaying the waveforms of the electrocardiac signals detected by the electrocardiac detector 30 on the display unit 52 of the external unit 50, and accurately make a diagnosis of the patient's condition (operation state of the heart). In addition, even in a case in which a detailed examination is to be carried out based on the results of the diagnosis, it is possible to speculate the state of the patient's heart from the body sounds heard by the physician and the electrocardiogram displayed on the display unit 52 of the external unit 50. As a result, it becomes possible to more accurately judge whether the detailed examination is required. In addition, the types of detailed examination to be carried out, and the accuracy with which these types of detailed examination are to be carried out, can be specified in advance, to thereby reduce the time required for the detailed examination and improve the examination accuracy of the required detailed examination.

Further, the external unit 50 may be formed by a terminal apparatus having a relatively large liquid crystal panel, for example, and the waveforms of the electrocardiogram can be displayed accurately in detail. Moreover, the external unit 50 time-sequentially stores the waveform data of the electrocardiogram, and for example, the electrocardiogram of 30 minutes before, the electrocardiogram of 20 minutes before, and the electrocardiogram of 10 minutes before can be successively displayed on the display unit 52 for the same patient by selecting the patient selection button 56. The physician can monitor the waveform data of the electrocardiogram stored in the external unit 50, and make a diagnosis of the patient by referring to the waveform data of the electrocardiogram in relation to a change in the condition of the patient. For this reason, the physician can readily confirm the change in the patient's heart condition by comparing the waveform data of the present electrocardiogram and the waveform data of the past electrocardiogram, and accurately diagnose the change in the patient's condition.

### [System Configuration of Diagnostic Apparatus]

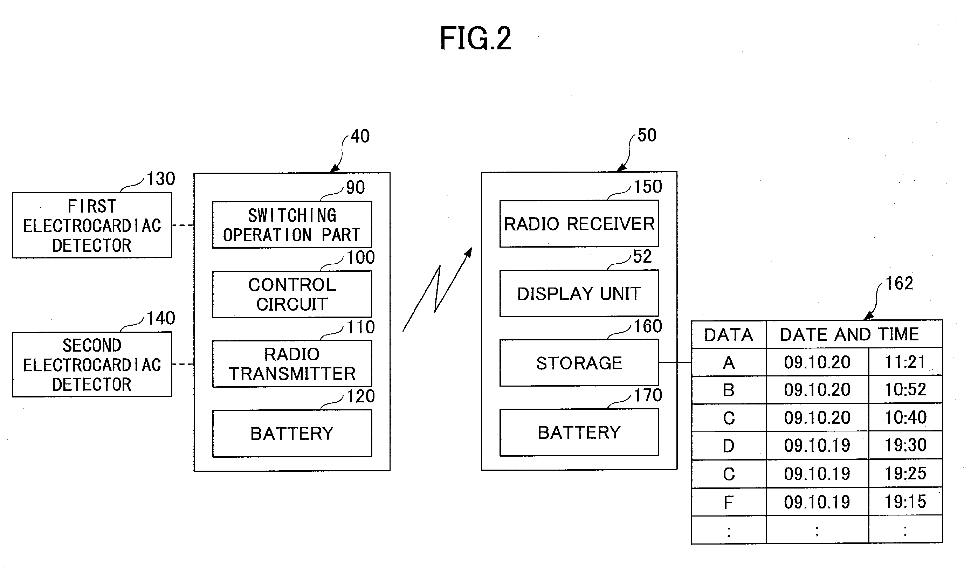

FIG. 2 is a block diagram illustrating a system configuration of the diagnostic apparatus. As illustrated in FIG. 2, the control unit 40 includes a switching operation part 90, a control circuit 100, a radio transmitter 110, and a battery 120. The switching operation part 90 switches the sound detector 60 to select one of the first and second detectors 62 and 64.

In addition, the first and second detectors 62 and 64 include first and second electrocardiac detectors 130 and 140 that are formed by the electrodes 31 through 33, respectively. When the control unit 40 switches the sound detector 60 and selects one of the first and second detectors 62 and 64, the control unit 40 reads the electrocardiac signals from the electrodes 31 through 33 that are provided on the selected one of the first and second electrocardiac detectors 130 and 140.

The control circuit 100 reads the electrocardiac signals detected by the first and second detectors 62 and 64, at a sampling frequency of 1 kHz, for example, and outputs to the radio transmitter 110 the waveform data that are obtained by performing a waveform shaping process to remove noise components included in the electrocardiac signals. The radio transmitter 110 converts the waveform data generated by the control circuit 100 into radio signals having a predetermined frequency, and transmits the radio signals.

The battery 120 includes a chargeable battery, and is charged by a charger that is provided on a support base or the like on which the stethoscope 20 is set when the stethoscope 20 is not in use, for example.

The external unit 50 is communicable with the control unit 30 using the radio signals. The external unit 50 includes, in addition to the display unit 52, a radio receiver 150, a storage 160, and a battery 170. The radio receiver 150 receives the radio signals from the control unit 40, demodulates the electrocardiac signals, and outputs the demodulated electrocardiac signals to the storage 160. The storage 160 time-sequentially stores the waveform data of the electrocardiac signals received by the radio receiver 150, together with the date and time when the measurement is made, in a database 162. The database 162 stores the waveform data of the electrocardiac signals, separately for each patient, in an order of the time of measurement. The waveform data of the electrocardiac signals of each patient can be read in the order of the time of measurement.

Accordingly, amongst the data stored in the database 162 of the storage 160, it is possible to first view the most recently stored data. When the data is selected from a storage list displayed on the display unit 52 in an order starting from the newest data, the data of the same patient can be displayed in the order of the time of measurement. In addition, in a case in which a plurality of patients are continuously diagnosed, and the patient selection buttons 56 of the external unit 50, corresponding to the plurality of patients, are selected, it is possible to display the waveform data of the electrocardiac signals of the plurality of patients on the display unit 52.

### [Configuration of Sound Detector]

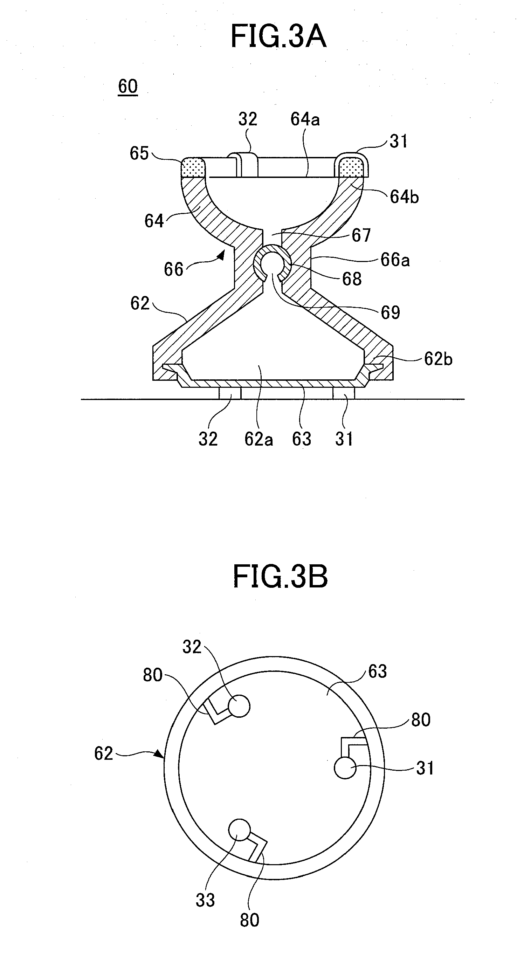

FIG. 3A is a longitudinal section of the sound detector in a state in which a diaphragm of the first detector makes contact with the patient's skin. As illustrated in FIG. 3A, the sound detector 60 includes a main body 66 having a hollow shape, and the first detector 62 for detecting the relatively high frequency (for example, 200 Hz or higher) is provided on one end (lower surface side in FIG. 3A) of the main body 66, and the second detector 64 for detecting the relatively low frequency (for example, 200 Hz or lower) is provided on the other end (upper end side in FIG. 3A) of the main body 66.

The first detector 62 has a tapered shape, and a diaphragm 63 made of a thin metal plate, a thin synthetic resin plate, or the like is fixed in an opening 62a. The diaphragm 63 has a disk shape, and an outer peripheral edge of the diaphragm 63 is held by an inner peripheral edge part 62b of the first detector 62 by calking.

In addition, the second detector 64 has a hemispherical shape, and a peripheral edge part 64b is covered by a resilient rubber member 65. An opening 64a of the second detector 64 communicates to the opening 62a of the first detector 62, via a penetration hole 67 that penetrates a constricted part 66a of the main body 66 in upward and downward directions.

A switching member 68 for switching sound detecting directions is inserted in a horizontal direction at an intermediate part of the penetration hole 67 so as to intersect the penetration hole 67. The switching member 68 is formed by a hollow pipe having a sound detecting hole 69 that forms a sound propagation path at an outer periphery of the hollow pipe.

The sound detector 60 can be rotated in the upward and downward direction about the switching member 68 as its center of rotation, in order to enable switching of the sound detecting direction from the first detector 62 to the second detector 64. The body sounds from the patient propagate through the sound detecting hole 69 in the switching member 68, the tube 70 of the stethoscope 20, and the ear tubes 72 and 74.

FIG. 3B is a bottom view illustrating an arrangement of the diaphragm and the electrodes of the sound detector. As illustrated in FIG. 3B, the diaphragm 63 that is fixed to the end part of the first detector 62 makes contact with the patient's skin, and vibrates when the body sounds from the patient propagate to the diaphragm 63. The diaphragm 63 has a characteristic that amplifies a high-frequency band of the body sounds in order to make the body sounds in the high-frequency band more audible. In addition, the electrodes 31 through 33 forming the first electrocardiac detector 130 are provided on the end surface (exposed surface) of the diaphragm 63 at angular intervals of 120 degrees along the circumferential direction.

The electrodes 31 through 33 of the first electrocardiac detector 130 are formed by a conductor material having a small electrical resistance, such as silver (Ag), silver chloride (AgCl), or the like. One of the electrodes 31 through 33 is a ground (GND) terminal, and the remaining two electrodes are detection terminals for detecting a potential difference caused by induced potentials. The electrodes 31 through 33 are connected to the control unit 40 via a plurality of wires 80.

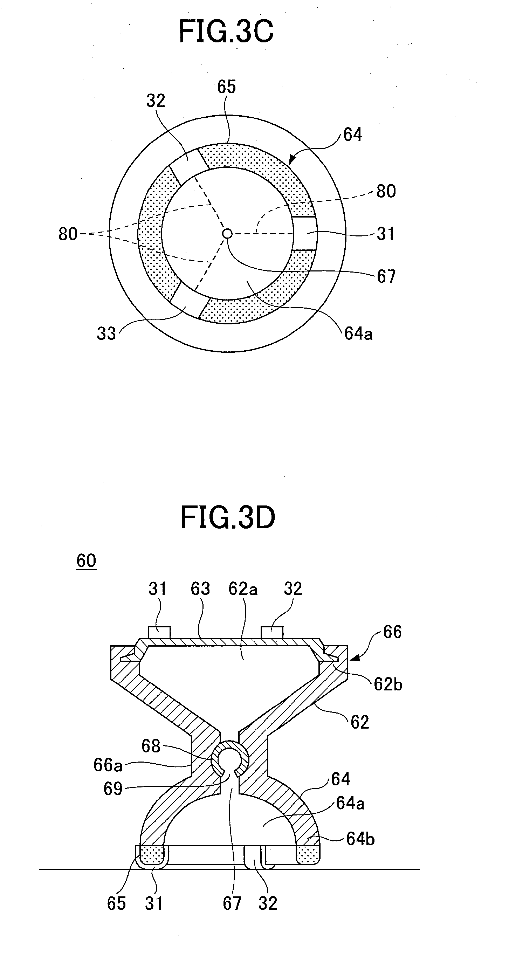

FIG. 3C is a plan view illustrating an arrangement of the rubber member and the electrodes at the peripheral edge part of the sound detector. As illustrated in FIG. 3C, the rubber member 65 having a ring-shape is fixed to the peripheral edge part 64b on the lower end side of the second detector 64. The rubber member 65 has an end part thereof formed in a hemispherical shape, to enable contiguous contact with the patient's skin. Because the rubber member 65 is resilient, the rubber member 65 absorbs the body sounds in the high-frequency band, and makes the body sounds in the low-frequency band more audible. In addition, the electrodes 31 through 33 forming the second electrocardiac detector 140 are provided on the end part (peripheral edge part) of the rubber member 65 at angular intervals of 120 degrees along the circumferential direction.

The electrodes 31 through 33 of the second electrocardiac detector 140 are formed by a conductor material having a small electrical resistance, such as silver (Ag), silver chloride (AgCl), or the like. One of the electrodes 31 through 33 is a ground (GND) terminal, and the remaining two electrodes are detection terminals for detecting a potential difference caused by induced potentials. The electrodes 31 through 33 are connected to the control unit 40 via a plurality of wires 80.

Each of the electrodes 31 through 33 of the first and second electrocardiac detectors 130 and 140 may be formed by a metal (for example, gold (Au)) other than silver (Ag) or silver chloride (AgCl), and having a small electrical resistance and a suitable corrosion resistance.

FIG. 3D is a longitudinal section of the sound detector in a state in which the rubber member of the sound detector makes contact with the patient's skin. As illustrated in FIG. 3D, in a case in which the body sounds in the low-frequency band are to be detected by the second detector 64, the sound detector 60 is rotated 180 degrees in the upward and downward directions about the switching member 68 at its center of rotation. In this case, the second detector 64 becomes located on the lower side by the switching of the state of the second detector 64, and the sound detecting hole 69 in the switching member 68 communicates to the opening 64a. Accordingly, the body sounds detected by the second detector 64 propagate through the sound detecting hole 69 in the switching member 68, the tube 70 of the stethoscope 20, and the ear tubes 72 and 74.

### [Detection of Electrocardiac Signals]

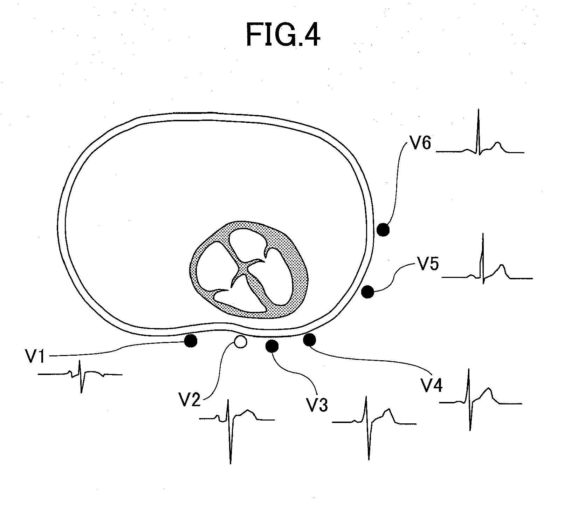

FIG. 4 is a cross sectional view illustrating detection points when the sound detector makes contact with a periphery of a chest part. As illustrated in FIG. 4, the first detector 62 or the second detector 64 of the sound detector 60 is made to contact the chest part of the patient, in order to detect the patient's body sounds. In addition, the electrodes 31 through 33 of the first and second electrocardiac detectors 130 and 140 are made to contact detection points V1 through V6, in order to detect electrocardiac signals associated with the patient's heart bets. Because the potential vector associated with the heart beats is different for each detection point, the potential difference measured at each detection point is also different for each detection point.

The electrocardiac signals (V1 induced signals) at the detection point V1 are induced signals for monitoring the heart mainly from a right ventricle side of the heart.

The electrocardiac signals (V2 induced signals) at the detection point V2 are induced signals for monitoring the heart from the right ventricle and left ventricular anterior wall side of the heart.

The electrocardiac signals (V3 induced signals) at the detection point V3 are induced signals for monitoring the heart from an interventricular septum and the left ventricular anterior wall of the heart.

The electrocardiac signals (V4 induced signals) at the detection point V4 are induced signals for monitoring the interventricular septum and a left ventricular anterior wall direction of the heart.

The electrocardiac signals (V5 induced signals) at the detection point V5 are induced signals for monitoring the left ventricular anterior wall and lateral wall of the heart.

The electrocardiac signals (V6 induced signals) at the detection point V6 are induced signals for monitoring the left ventricular lateral wall of the heart.

By successively changing the contact position of the sound detector 60 to each of the detection points V1 through V6, it is possible to measure the electrocardiac signals at each of the detection points V1 through V6.

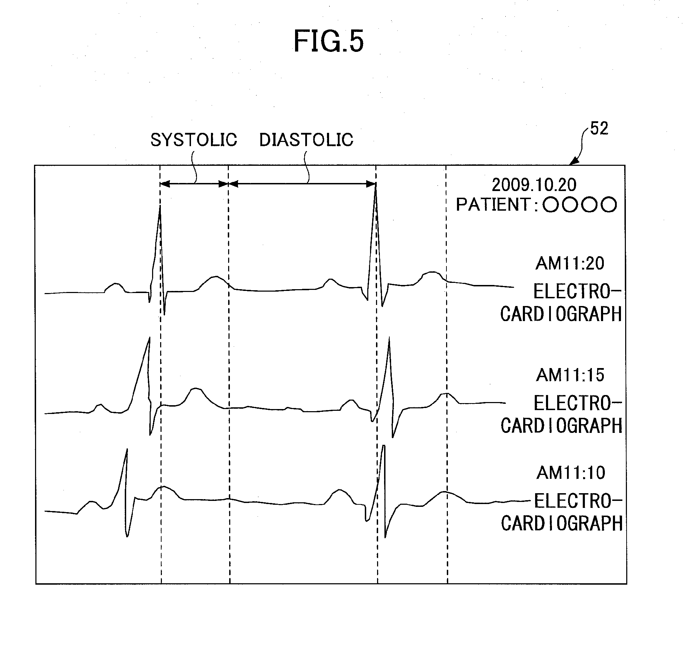

FIG. 5 is a diagram illustrating waveforms displayed on the display unit of the external unit. As illustrated in FIG. 5, the display unit 52 of the external unit 50 displays the waveforms of the electrocardiac signals detected at each of the detection points V1 through V6, in order to display the most recent measured data in parallel from the top. Hence, the physician can confirm the waveforms of the electrocardiac signals displayed on the display unit 52 of the external unit 50, while listening to the patient's body sounds (cardiac sound) by the stethoscope 20, and make a diagnosis on the operation state of the patient's heart.

In addition, from the waveforms of the electrocardiac signals displayed on the display unit 52 of the external unit 50, it is possible to make a diagnosis on whether an abnormality is generated in the state of a systolic blood pumping operation or a diastolic blood suction operation. Further, in a case in which arhythmia exists, it is possible to confirm the state of arhythmia, the state of tachycardia or bradycardia, or the like from the waveforms of the electrocardiac signals.

### [Example of Usage of Diagnostic Apparatus]

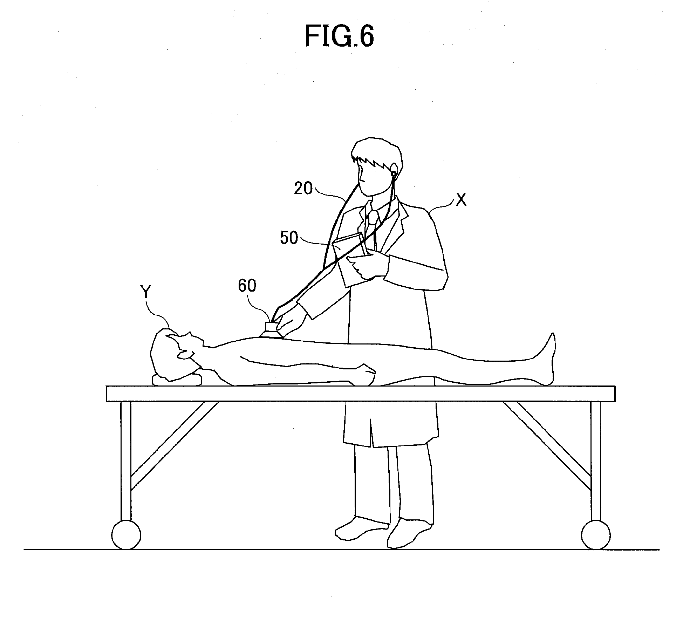

FIG. 6 is a diagram illustrating a state in which the physician makes a diagnosis using the diagnostic apparatus of the present invention. As illustrated in FIG. 6, a physician X places the first detector 62 or the second detector 64 of the sound detector 60 to make contact with the chest part of a patient Y, and makes a diagnosis on the operation state of the heart of the patient Y by confirming the waveforms of the electrocardiac signals displayed on the display unit 52 of the external unit 50 while listening to the respiratory sound and the cardiac sound of the patient Y. In this case, the physician X may move by carrying the stethoscope 20 and the external unit 50, and thus, the physician X can make an accurate diagnosis on the operation state of the heart of the patient Y even at a location (for example, disaster site) other than an examination room of a hospital.

In addition, when making the conventional diagnosis based on the body sounds such as the respiratory sound, a diagnosis can be made while confirming the electrocardiogram. For this reason, an accurate judgment may be made on whether a more detailed examination is required based on a high-precision electrocardiogram using the 12-lead electrocardiograph or the like.

Furthermore, at the disaster site or the like, a diagnostic time can be reduced by improving a diagnostic efficiency of the physician X. Even when making the diagnosis of a large number of patients Y at the time of a disaster or the like, the diagnosis of the state of the heart of the patients Y can be made with ease, and a priority order of treatment (triage) can be determined depending on the state of the heart of a large number of patients Y at the time of the disaster or the like.

Next, a description will be given of modifications.

### [System Configuration in Modification 1]

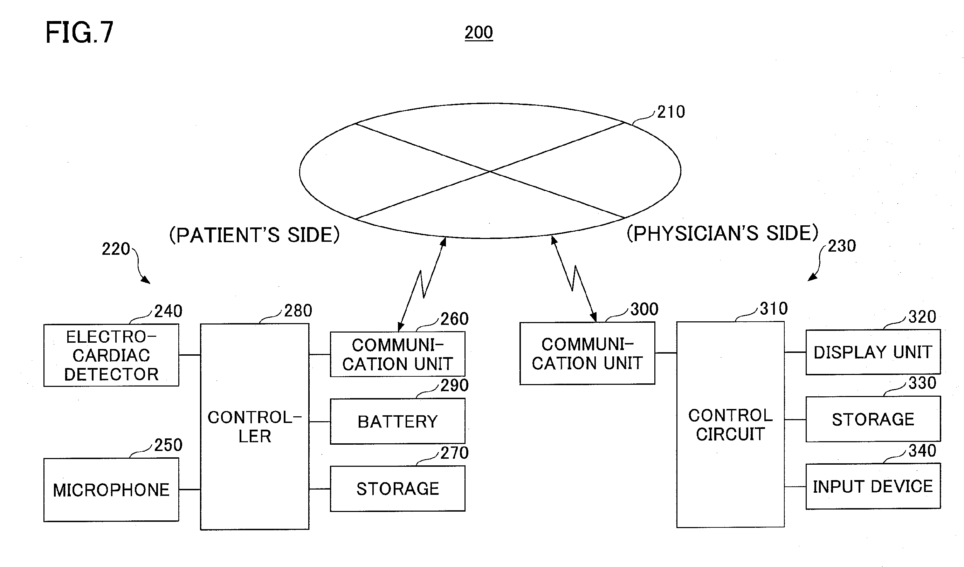

FIG. 7 is a block diagram illustrating a system configuration of a modification 1. As illustrated in FIG. 7, a diagnostic apparatus 200 in this modification 1 may transmit and receive image data via the Internet 210, and form a diagnostic system in which a patient who is located at an isolated island, a mountain area, or the like can be diagnosed by a physician in a hospital located at a remote location.

The diagnostic apparatus 200 includes a stethoscope unit 220 used by the patient, and a physician's side terminal apparatus 230 that is set up in a hospital or the like at the remote location. The stethoscope unit 220 is compact and portable, and may be operated by the patient. The stethoscope unit 220 includes an electrocardiac detector 240, a microphone 250, a communication unit 260, a memory (RAM) 270, a controller 280, and a battery 290. The communication unit 260 converts the electrocardiac signals detected by the electrocardiac detector 240 and cardiac sound signals detected by the microphone 250 into radio signals, and transmits the radio signals to the Internet 210 via a communication apparatus such as a router or the like. The radio signals of the electrocardiac signals detected by the electrocardiac detector 240 and the cardiac sound signals detected by the microphone 250 are transmitted via the Internet 210 to the physician's side terminal apparatus 230 that is set up at a remote location.

The physician's side terminal apparatus 230 is formed by a personal computer or the like, for example, and includes a communication unit 300, a control circuit 310, a display unit 320, a storage 330, and an input device 340. The communication unit 300 is connectable to the Internet 210 via a public network. The physician's side terminal apparatus 230 stores in the storage 330 and displays on the display unit 320, waveform data of the electrocardiac signals detected by the electrocardiac detector 240 and the cardiac sound signals detected by the microphone 250, received from the stethoscope unit 220.

The input device 340 includes a keyboard, a mouse, or the like, and may be operated by the physician to display on the display unit 320 arbitrary data selected from data of each patient stored in the storage 330 and data of the electrocardiac signals and cardiac sound signals received from the stethoscope unit 220.

### [Stethoscope Unit of Modification 1]

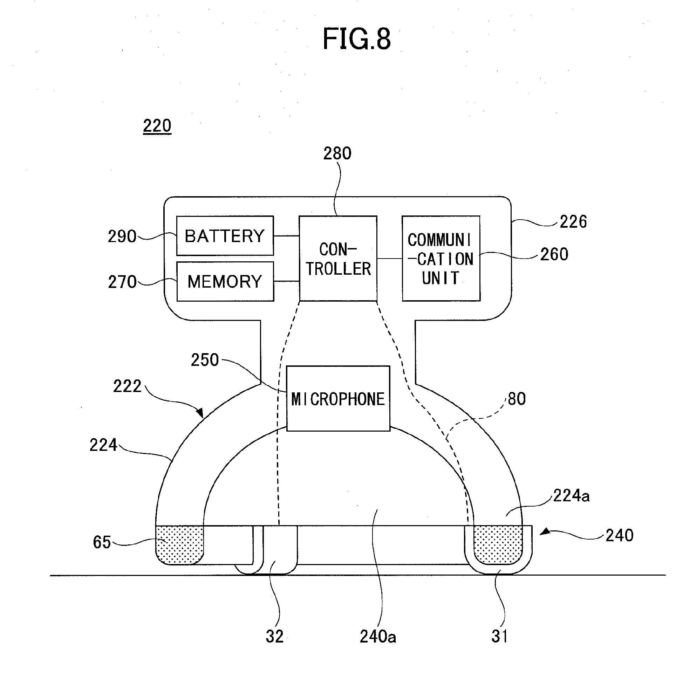

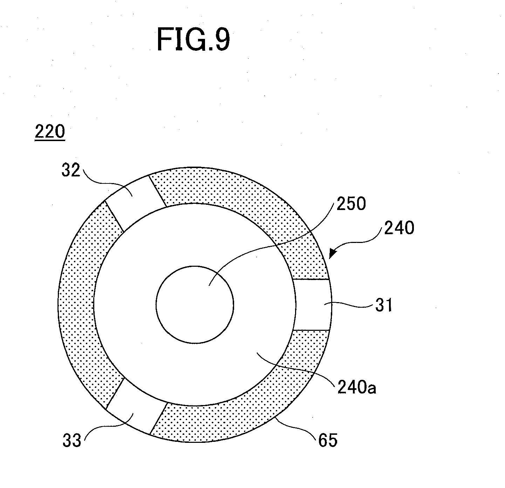

FIG. 8 is a longitudinal section of the stethoscope unit of the modification 1. FIG. 9 is a bottom view illustrating an end surface of the stethoscope unit of the modification 1. As illustrated in FIGs. 8 and 9, the stethoscope unit 220 includes a main body 222, and a bell-shaped detector 224 provided on a lower part of the main body 222. The bell-shaped detector 224 has a ringshaped rubber member 65 fixed to a lower end peripheral edge part 224a of the main body 222.

The electrodes 31 through 33 forming the electrocardiac detector 240 are provided on the outer periphery (peripheral edge part) of the rubber member 65 at angular intervals of 120 degrees along the circumferential direction.

In addition, the microphone 250 is provided at a center of an opening 240a in the electrocardiac detector 240. The microphone 250 detects the body sounds (including respiratory sound and cardiac sound) propagating through the patient's skin, when the electrodes 31 through 33 of the stethoscope unit 240 make contact with the patient's skin, and outputs signals according to the detected body sounds.

The stethoscope unit 220 has a circular handle part 226 at the upper part of the main body 222, in order to facilitate handling of the stethoscope unit 220. The communication unit 260, the memory 270, the controller 280, and the battery 290 are accommodated within the handle part 226.

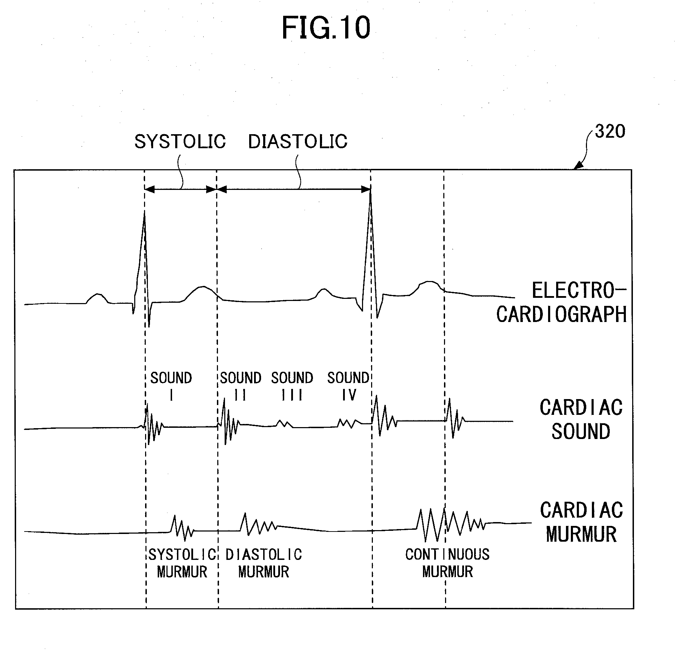

FIG. 10 is a diagram illustrating waveforms of the signals detected by the stethoscope unit of the modification 1. When the controller 280 shapes the waveforms of the electrocardiac signals detected by the electrocardiac detector 240 of the stethoscope unit 220 and the signals of the cardiac sound and cardiac murmur detected by the microphone 250 of the stethoscope unit 220, and the shaped waveforms are transmitted from the communication unit 260, the waveforms of the electrocardiac signals, the cardiac sound, and the cardiac murmur received from the stethoscope unit 220 are displayed on the display unit 320 of the physician's side terminal apparatus 230 that is provided at the remote location, as illustrated in FIG. 10. As a result, the physician at the remote location can make a diagnosis on the operation state of the patient's heart based on the waveforms of the electrocardiac signals, the cardiac sound, and the cardiac murmur that are displayed on the display unit 320 of the physician's side terminal apparatus 230.

### [Example of Usage of Modification 1]

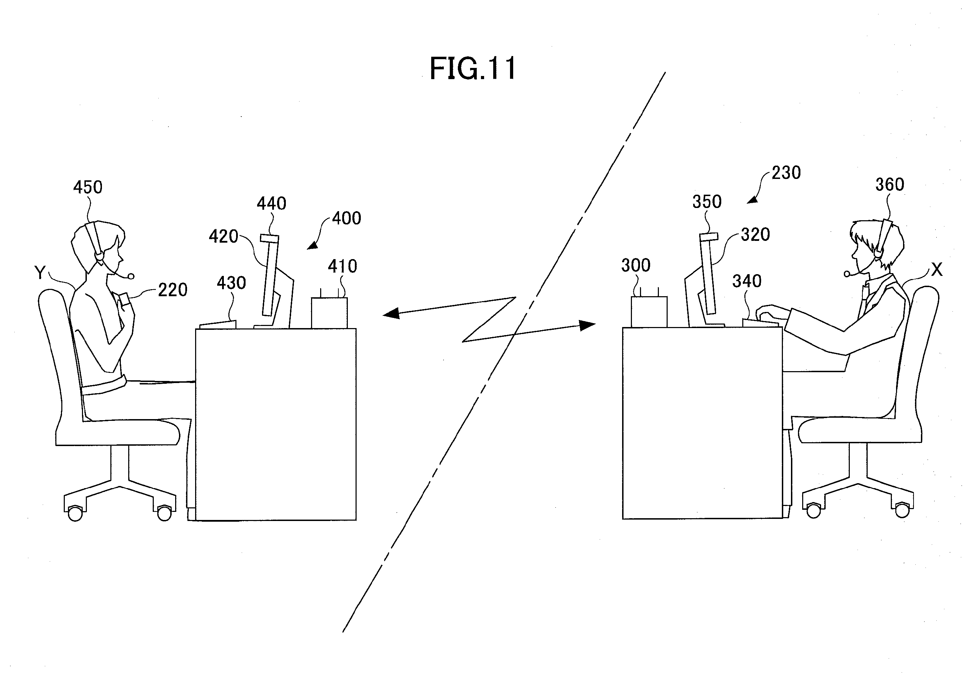

FIG. 11 is a diagram schematically illustrating a state in which the physician makes the diagnosis using the diagnostic apparatus of the modification 1. As illustrated in FIG. 11, the patient Y sits in front of a patient's side terminal apparatus 400, holds the handle part 226 of the stethoscope unit 220, and places the electrocardiac detector 240 against his or her chest to make contact therewith. In this modification, the waveform data of the electrocardiac signals, the cardiac sound, and the cardiac murmur transmitted from the stethoscope unit 220 are temporarily stored in a storage of the patient's side terminal apparatus 400.

The patient's side terminal apparatus 400 has a configuration similar to that of the physician's side terminal apparatus 230, and includes a communication unit 410, a display unit 420, an input device 430, and a CCD camera 440. The patient's side terminal apparatus 400 transmits to the physician's side terminal apparatus 230 the waveform data of the electrocardiac signals, the cardiac sound, and the cardiac murmur transmitted from the stethoscope unit 220, via the communication unit 410 and the public network or the Internet. In addition, the patient Y wears a headphone with microphone, 450, on his or her head so that the patient Y may make conversation with the physician X.

A CCD camera 350 is also provided on the display unit 320 of the physician's side terminal apparatus 230. The physician X wears a headphone with microphone, 360, on his or her head so that the physician X may make conversation with the patient Y. In addition, an image of the patient Y picked up by the CCD camera 440 is displayed in real-time on the display unit 320 of the physician's side terminal apparatus 230. Similarly, an image of the physician X picked up by the CCD camera 350 is displayed in real-time on the display unit 420 of the patient's side terminal apparatus 400.

Accordingly, conversation is possible between the physician X and the patient Y, while monitoring faces of each other displayed on the display units 320 and 420. For this reason, the physician X can ask questions to and receive response from the patient Y using the headphones with microphones, 360 and 450. The physician X can examine face expressions on the patient Y by monitoring the displayed image of the patient Y, and instruct the contact position of the stethoscope unit 220 to the patient Y, using the display units 320 and 420.

On the other hand, in a case in which the patient Y is unable to make conversation with the physician X, a family member or a friend helping the patient Y may operate the stethoscope 220 and also respond to the questions from the physician X.

Therefore, even in a case in which the physician X and the patient Y are at distant locations from each other, the physician X can carry out the examination by receiving responses to questions and by listening to the body sounds of the patient Y. The physician X can thus make a diagnosis on the condition of the patient Y using the waveform data of the electrocardiac signals, the cardiac sound, and the cardiac murmur transmitted from the stethoscope unit 220.

### [Configuration of Modification 2]

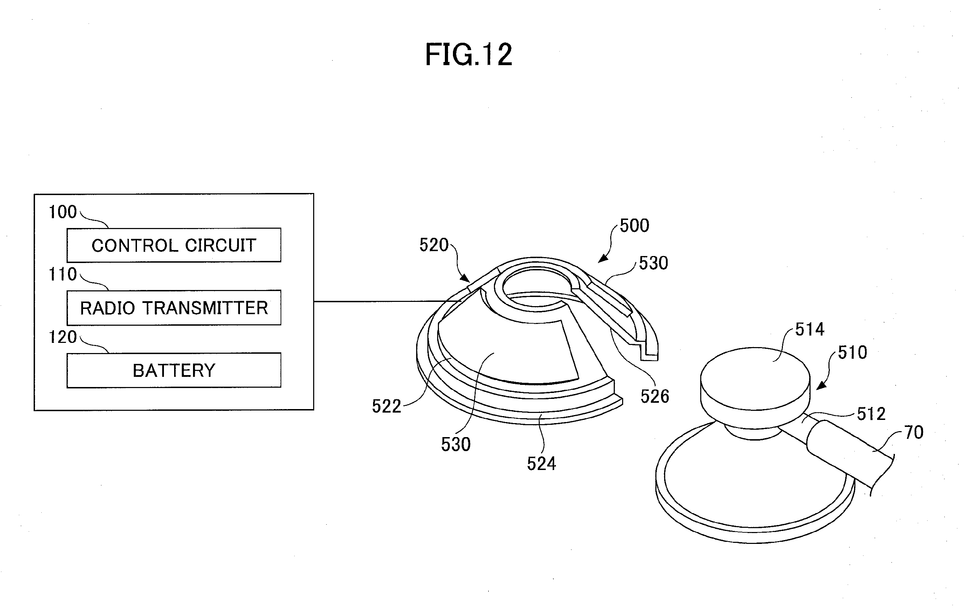

FIG. 12 is a perspective view illustrating a state in which the electrocardiac unit of a modification 2 is separated from the stethoscope. As illustrated in FIG. 12, an electrocardiac unit 500 in this modification 2 is detachably connectable to a sound detector 510 of an existing stethoscope. In addition, the electrocardiac unit 500 includes a mounting base 520, and a control unit 530 that is mounted on the mounting base 520.

The control unit 530 includes a control circuit 100, a radio transmitter 110, and a battery 120, similarly to the control unit 40 illustrated in FIG. 2. The control circuit 100 and the radio transmitter 110 may be formed as a package in which each electronic component made up of an IC chip is mounted on a flexible printed circuit. The battery 120 may be formed by a thin, small, and light button-shaped battery (mercury battery) or the like.

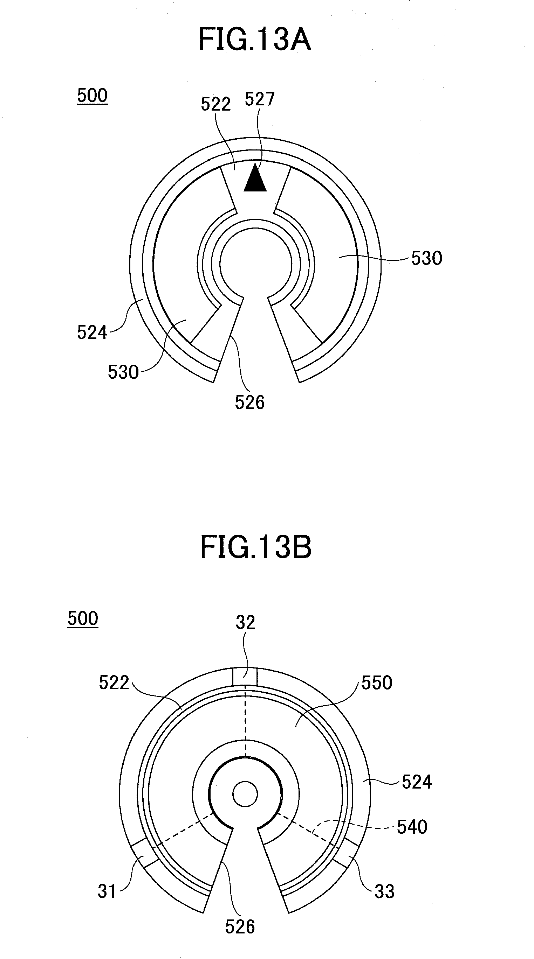

FIG. 13A is a plan view of the stethoscope unit of the modification 2 viewed from above. As illustrated in FIG. 13A, a mounting base 520 of the electrocardiac unit 500 may be molded from a flexible resin, such as silicon or the like, and includes a circular cone-shaped part 522, an electrocardiac detector 524 projecting in a horizontal direction from an outer peripheral side of the circular cone-shaped part 522, and a slit 526.

The slit 526 is formed to extend in the upward and downward directions along the circular cone-shaped part 522 and the electrocardiac detector 524. In addition, a width of the slit 526 is adjustable in a circumferential direction so that this width may be enlarged in the circumferential direction when connecting the mounting base 520 to the sound detector 510 of the stethoscope. After the mounting base 520 of the electrocardiac unit 500 is connected to the sound detector 510 of the stethoscope, the width of the slit 526 returns to its original narrower width due to the resiliency of the mounting base 520 itself, and thus, the circular cone-shaped part 522 of the mounting base 520 assumes a state in contact with a circular cone-shaped part of the sound detector 510.

The control unit 530 is mounted on an upper surface side of the circular cone-shaped part 522. The control unit 530 in this embodiment is segmented into two packages due to the set-up space available. One package includes the IC chip of the control circuit 100 and the radio transmitter 110, while the other package accommodates the battery 120.

A triangular mark 527 is provided on the upper surface side of the circular cone-shaped part 522, as a marker indicating the vector direction of the electrocardiac signals to be measured at the electrode positions. In a state in which the electrocardiac unit 500 is connected to the sound detector 510 of the stethoscope, the direction of the electrode 32 is indicated by the triangular mark 527.

FIG. 13B is a bottom view of the stethoscope unit of the modification 2 viewed from below. As illustrated in FIG. 13B, the electrocardiac detector 524 is provided on an outer peripheral edge part of the mounting base 520. Similarly as in the case of the embodiment described above, the electrodes 31 through 33 for detecting potentials associated with heart beats are arranged at angular intervals of 120 degrees on the electrocardiac detector 524. One of the electrodes 31 through 33 is the ground (GND) terminal, and the remaining two electrodes are the detection terminals for detecting the potential difference caused by induced potentials. The electrodes 31 through 33 are connected to the control unit 530 via a plurality of wires 540 that are formed on a lower surface side of the mounting base 520.

Each of the electrodes 31 through 33 of the electrocardiac detector 524 is provided on the outer peripheral edge part that is formed to be sufficiently wide, so that a contact area can be secured. Each of the electrodes 31 through 33 and the plurality of wires 540 may be formed on the lower surface side of the mounting base 520 by plating or the like. A non-slip sheet 550, which makes contiguous contact with the surface of the electrocardiac detector 510, is adhered on the lower surface of the circular cone-shaped part 522 of the mounting base 520.

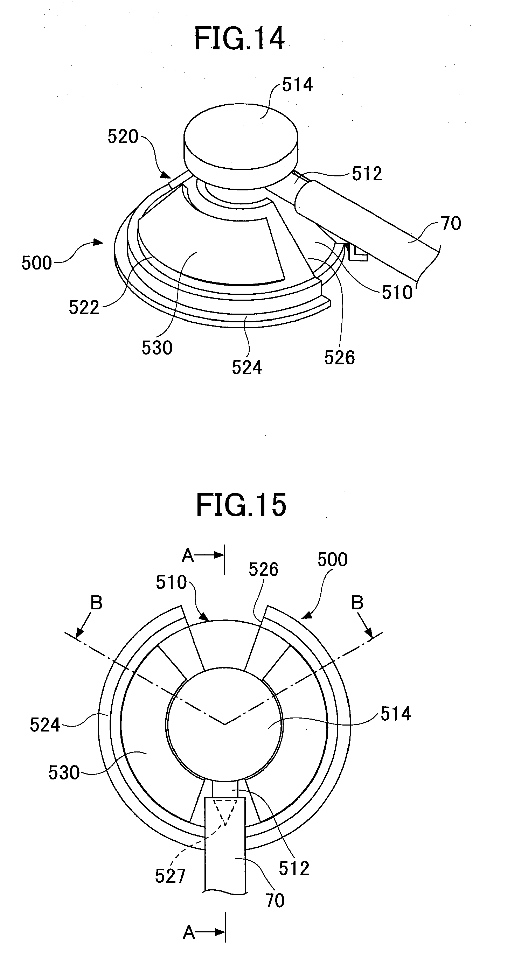

FIG. 14 is a perspective view illustrating a state in which the electrocardiac unit of the modification 2 is connected to the stethoscope. As illustrated in FIG. 14, in a state in which the electrocardiac unit 500 is connected to the sound detector 510 of the stethoscope, both edge parts defining the slit 526 of the mounting base 520 hold the tube connecting part 512 that projects sideward from the sound detector 510. The tube connecting part 512 is provided to connect the tube 70, however, the tube connecting part 512 also functions as a stopper that prevents the electrocardiac unit 500 from rotating.

A large-diameter part 514 provided on the upper part of the sound detector 510 of the stethoscope functions as a handle part that may be held by the physician when placing the sound detector 510 to make contact with the patient.

FIG. 15 is a plan view illustrating the state in which the stethoscope unit of the modification 2 is connected to the stethoscope. As illustrated in FIG. 15, in the state in which the electrocardiac unit 500 is connected to the sound detector 510 of the stethoscope, the mounting base 520 is rotated approximately 180 degrees, so that the triangular mark 527 provided on the upper surface side of the circular cone-shaped part 522 and indicating the vector direction of the electrocardiac signals assumes a position (on the physician's side) along the direction in which the tube 70 extends. As a result, when viewed from the physician's side, the mark 527 is located at the position on the near side (physician's side), and it can be seen that the electrode 32 provided at the position corresponding to the mark 527 is located on the side of the tube 70 that becomes the vector direction of the electrocardiac signals.

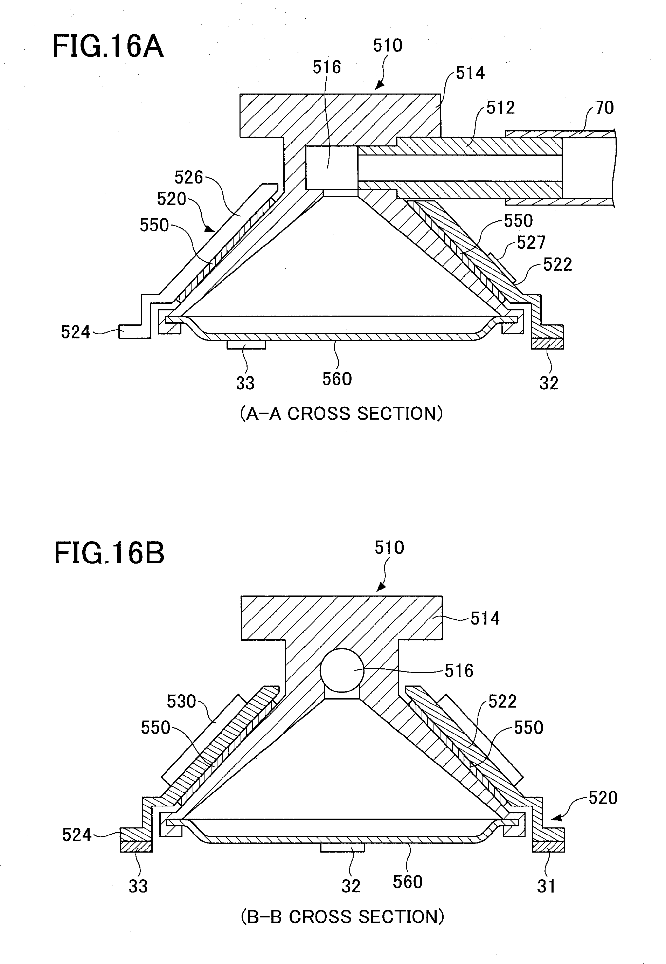

FIG. 16A is a longitudinal section along a line A-A in FIG. 15. FIG. 16B is a longitudinal section along a line B-B in FIG. 15. As illustrated in FIGs. 16A and 16B, the sound detector 510 forms a chest piece of the existing stethoscope, and the diaphragm 63 is fixed at the bottom opening by calking. In addition, a sound propagation path 516 is provided inside the sound detector 510. Vibration sounds from the diaphragm 63, that is vibrated by the propagating body sounds, propagate through the sound propagation path 516.

In the state in which the electrocardiac unit 500 is connected to the sound detector 510 of the stethoscope, the slit 526 is formed below the tube connecting part 512 due to the resiliency of the mounting base 520, to thereby enable the electrocardiac unit 500 to rotate in the circumferential direction with respect to the sound detector 510. In addition, because the non-slip sheet 550 adhered on the lower surface side of the circular cone-shaped part 522 of the mounting base 520 has a surface with a high coefficient of friction, the circular cone-shaped part 522 is maintained in a state in contact with a sloping surface of the sound detector 510.

The lower peripheral edge part of the sound detector 510 holds an outer peripheral part of a diaphragm 560. A lower surface of the diaphragm 560 is formed to be located at a position lower than that of the electrocardiac detector 524 and higher than that of a lower surface side contact surface of each of the electrodes 31 through 33.

Because each of the electrodes 31 through 33 provided on the outer peripheral edge part of the mounting base 520 projects downwardly to a position lower than the lower surface of the diaphragm 560, when the diaphragm 560 is made to contact the patient's skin to detect the body sounds, the electrodes 31 through 33 make contact with the patient's skin to enable detection of the potentials associated with the heart beats. The electrocardiac signals measured by the electrodes 31 through 33 are converted into radio signals by the control unit 530, in a manner similar to the conversion performed by the control unit 40 described above, and the radio signals are transmitted to the external unit 50. For this reason, the physician can make an accurate diagnosis on the patient's condition (operation state of the heart) by placing the sound detector 510 of the stethoscope to make contact with the patient in order to listen to the patient's body sounds (including cardiac sound and respiratory sound), while causing the display unit 52 of the external unit 50 to display the waveforms of the electrocardiac signals detected by the electrodes 31 through 33. In addition, even in a case in which a detailed examination is to be carried out based on the results of the diagnosis, it is possible to speculate the state of the patient's heart from the body sounds heard by the physician and the electrocardiogram displayed on the display unit 52 of the external unit 50. As a result, it is possible to reduce the time required for the detailed examination and improve the examination accuracy of the detailed examination, by specifying in advance the parts of the heart to be subjected to the detailed examination.

Accordingly, by connecting the electrocardiac unit 500 in this modification 2 to the sound detector 510 of the stethoscope, the electrocardiac signals can be measured while listening to the patient's body sounds even by use of the existing stethoscope, and the diagnosis of the state of the patient's heart becomes possible based on the electrocardiograph displayed on the display unit 52 of the external unit 50.

### [Configuration of Modification 3]

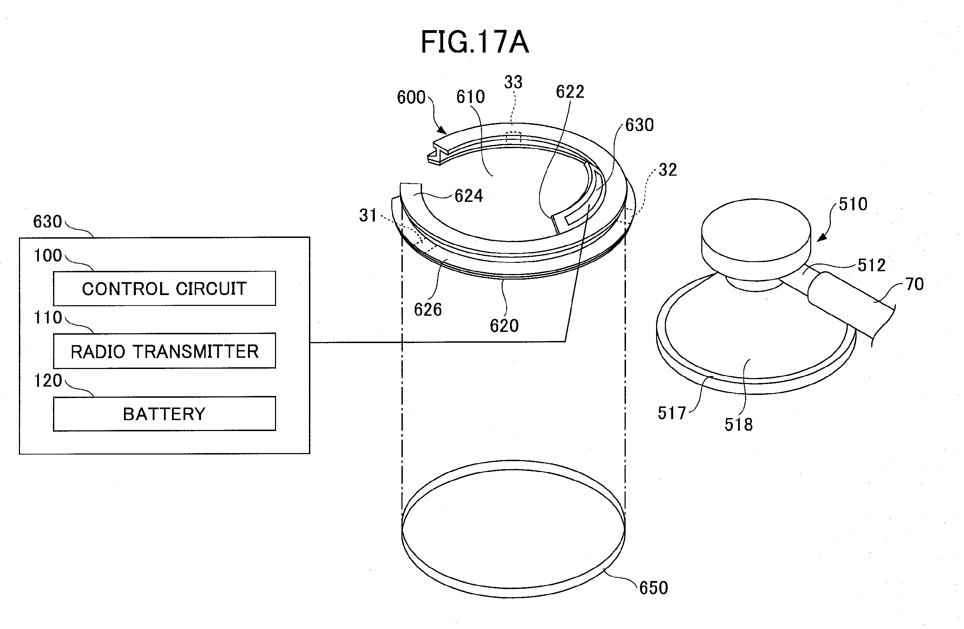

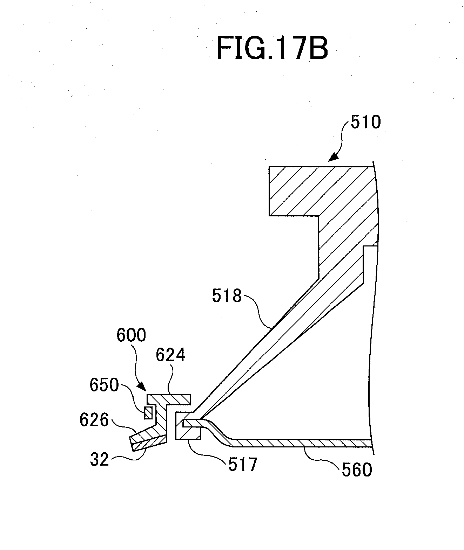

FIG. 17A is a perspective view illustrating a state in which an electrocardiograph unit of a modification 3 is separated from the stethoscope. FIG. 17B is a longitudinal section illustrating a cross section of a part of the stethoscope unit of the modification 3.

As illustrated in FIGs. 17A and 17B, an electrocardiac unit 600 in this modification 3 is detachably connectable to a sound detector 510 of an existing stethoscope. In addition, the electrocardiac unit 600 includes a mounting base 620, and a control unit 630 that is mounted on the mounting base 620. A method of connecting the electrocardiac unit 600 to the sound detector 510 may be similar to the method used in the case of the modification 2 described above.

The control unit 630 includes a control circuit 100, a radio transmitter 110, and a battery 120, similarly to the control unit 40 described above (refer to FIG. 2).

The mounting base 620 includes a mounting part 622 on which the control unit 630 is mounted, a stepped fitting part 624 that fits to an outer peripheral edge part 517 of the sound detector 510, and a flange part 626 having the electrodes 31 through 33 arranged on a lower surface side thereof. The mounting base 620 is formed to an approximate ring shape, and includes a mounting part 622 that projects and slopes towards the inner peripheral side, and a slit 640 located on the opposite side (180-degree direction) from the mounting part 622.

The mounting part 622 is provided in a trapezoidal shape at an inner peripheral intermediate part of the mounting base 620, and not for the entire circumference of the mounting base 620. An opening is formed virtually on the entire inner peripheral side of the mounting base 620. For this reason, when connecting the electrocardiac unit 600 to the sound detector 510, the stepped fitting part 624 can be fitted on the outer peripheral edge part 517 of the sound detector 510 by a relatively simple operation of widening the width of the slit 640.

When connecting the electrocardiac unit 600 to the sound detector 510, the slit 640 is arranged to oppose a part where the tube 70 is not provided, and after fitting the stepped fitting part 624 on the outer peripheral edge part 517 of the sound detector 510, the mounting base 620 is rotated in the circumferential direction in order to adjust the control unit 630 to a position below the tube 70. Because the electrode 32 is provided on the lower surface of the flange part 626 located below the control unit 630, this electrode 32 is located at a position below the tube 70 that becomes the vector direction of the electrocardiac signals.

Thereafter, a cable tie 650 is made to make contiguous contact with an outer periphery of the stepped fitting part 624, in order to fasten the stepped fitting part 624 from the outer peripheral side thereof. As a result, the width of or the distance between both edge parts defining the slit 640 is reduced, and the stepped fitting part 624 is held in a state making contiguous contact with the outer peripheral edge part 517 of the sound detector 510. For example, the cable tie 650 may be formed by a resilient member made of elastomer, or a resin belt that can be fastened.

In the state in which the electrocardiac unit 600 is connected to the sound detector 510, virtually all of the circular cone-shaped part 518 of the sound detector 510 is exposed, because the mounting part 622 is provided only at a part of the inner periphery of the mounting base 620. For this reason, when the physician holds the sound detector 510 to make the diagnosis, a diagnosis operation similar to that when using the existing stethoscope (having no electrocardiac unit 600) is possible. In other words, the electrocardiac signals can be measured without requiring the physician to perform an unfamiliar or uncomfortable operation of the stethoscope.

### DESCRIPTION OF REFERENCE NUMERALS

- 10: Diagnostic Apparatus
- 20: Stethoscope
- 30: Electrocardiac Detector
- 31 - 33: Electrodes
- 40: Control Unit
- 50: External Unit
- 52: Display Unit
- 54: Operation Part
- 56: Patient Selection Buttons
- 60: Electrocardiac Detector
- 62: First Detector
- 63: Diaphragm
- 64: Second Detector
- 65: Rubber Member
- 66: Main Body
- 68: Switching Member
- 69: Sound Detecting Hole
- 70: Tube
- 72, 74: Ear Tubes
- 76, 78: Ear Tips
- 80: Wires
- 90: Switching Operation Part
- 100: Control Circuit
- 110: Radio Transmitter
- 120: Battery
- 130: First Electrocardiac Detector
- 140: Second Electrocardiac Detector
- 150: Radio Receiver
- 160: Storage
- 162: Database
- 170: Battery
- 200: Diagnostic Apparatus
- 210: Internet
- 220: Stethoscope Unit
- 222: Main Body
- 224: Bell-shaped Detector
- 226: Handle Part
- 230: Physician's Side Terminal Apparatus
- 240: Electrocardiac Detector
- 250: Microphone
- 260, 300, 410: Communication Unit
- 270: Memory (RAM)
- 280: Controller
- 290: Battery
- 320, 420: Display Unit
- 330: Storage
- 340, 430: Input Device
- 350, 440: CCD Camera
- 360, 450: Headphone With Microphone
- 400: Patient's Side Terminal Apparatus
- 500, 600: Electrocardiac Unit
- 510: Sound Detector
- 512: Tube Connecting Part
- 514: Large-Diameter Part
- 516: Sound Propagation Path
- 517: Outer Peripheral Edge Part
- 518: Circular Cone-shaped Part
- 520: Mounting Base
- 530, 630: Control Unit
- 522: Circular Cone-Shaped Part
- 524: Electrocardiac Detector
- 526, 640: Slits
- 527: Mark
- 540: Wires
- 550: Non-Slip Sheet
- 620: Mounting Base
- 622: Mounting Part
- 624: Stepped Fitting Part
- 626: Flange Part
- 650: Cable Tie

## Claims

1. A diagnostic apparatus comprising:
a stethoscope including a sound detector that detects body sounds, a tube having one end thereof communicating to the sound detector, and a pair of ear tubes branching from another end of the tube, and configured to detect the body sounds from end parts of the pair of ear tubes;
an electrocardiac detector, provided on the sound detector, and configured to detect potentials associated with heart beats;
a control unit, provided on the stethoscope, and configured to convert the potentials detected by the electrocardiac detector into radio signals and transmit the radio signals; and
an external unit, provided separately from the stethoscope, and configured to receive the radio signals transmitted from the control unit and display waveforms based on the radio signals.

2. The diagnostic apparatus as claimed in claim 1, wherein the electrocardiac detector and the control unit are provided on a mounting base that is detachably mounted on the sound detector.

3. The diagnostic apparatus as claimed in claim 1 or 2, wherein the control unit includes
a radio transmitter configured to convert the potentials detected by the electrocardiac detector into the radio signals; and
a battery configured to supply power to the radio transmitter.

4. The diagnostic apparatus as claimed in any of claims 1 to 3, wherein the external unit is formed by a portable terminal apparatus including
a radio receiver configured to receive the radio signals transmitted from the radio transmitter;
a storage configured to store a change in the potentials from the radio receiver; and
a display unit configured to display the waveforms based on the change in the potentials.

5. The diagnostic apparatus as claimed in any of claims 1 to 4, wherein the sound detector includes
a first detector having a diaphragm configured to detect a high frequency of the body sounds; and
a second detector having a rubber member, provided on a peripheral edge part of the sound detector, and configured to detect a low frequency of the body sounds,
wherein the first detector and the second detector are selectable, and
wherein the electrocardiac detector is provided on each of the first detector and the second detector.

6. A diagnostic apparatus comprising:
a stethoscope unit including a sound detector configured to detect body sounds; and
a terminal apparatus, provided separately from the stethoscope unit, and configured to receive radio signals transmitted from the stethoscope unit and display waveforms based on the radio signals and cardiac sound signals,
wherein the stethoscope unit includes an electrocardiac detector configured to detect potentials associated with heart beats, a microphone configured to detect cardiac sound, and a radio transmitter configured to convert the potentials detected by the electrocardiac detector and the cardiac sound detected by the microphone into radio signals and transmit the radio signals to the terminal apparatus.

7. The diagnostic apparatus as claimed in claim 6, wherein the stethoscope unit includes
a storage configured to store the potentials detected by the electrocardiac detector and the cardiac sound signals detected by the microphone; and
a battery configured to supply power to the radio transmitter.

8. The diagnostic apparatus as claimed in claim 6 or 7, wherein the terminal apparatus includes
a communication unit including a radio receiver configured to receive the radio signals of the potentials and the cardiac sound signals transmitted from the radio transmitter of the stethoscope unit;
a storage configured to store a change in the potentials and the cardiac sound signals from the communication unit; and
a display unit configured to display waveforms based on the change in the potentials detected by the electrocardiac detector and the cardiac sound signals detected by the microphone.
